# EUROPÄISCHE PATENTSCHRIFT

(11) **EP 0 672 087 B1**
(45) Veröffentlichungstag und Bekanntmachung des Hinweises auf die Patenterteilung: **21.04.1999**
(21) Anmeldenummer: 94928793.2
(22) Anmeldetag: 21.09.1994
(51) Int. Cl.: C09B 29/00, G01N 31/22

(54) **INDIKATOREN ZUR BESTIMMUNG DER PROTONENKONZENTRATION**
INDICATORS TO DETERMINE PROTON CONCENTRATION
INDICATEURS PERMETTANT DE DETERMINER LA CONCENTRATION EN PROTONS

(30) Priorität: 02.10.1993 DE 4333696
(43) Veröffentlichungstag der Anmeldung: 20.09.1995
(73) Patentinhaber: MERCK PATENT GmbH, 64293 Darmstadt (DE)
(72) Erfinder: FISCHER, Wolfgang, D-64283 Darmstadt (DE); BAUM, Sylvia, D-64347 Griesheim (DE); HARTIG, Thorsten, D-64846 Gro -Zimmern (DE); SCHLEEHAHN, Michael, D-64678 Lindenfels (DE)
(86) Internationale Anmeldenummer: EP9403156
(87) Internationale Veröffentlichungsnummer: WO9509894

(56) Entgegenhaltungen:
- EP-A- 0 287 909

## Beschreibung

Die Erfindung betrifft Indikatoren zur Bestimmung der Protonenkonzentration stark alkalischer wäßriger Lösungen.

Der übliche, täglich im Labor oder auch in der Umwelt benutzte pH-Bereich geht von pH 0 bis pH 14. Darauf sind auch die üblichen pH-Papiere, pH-Indikatorstäbchen und pH-Meter eingestellt; sie messen nur in diesem Bereich. In vielen Fällen ist es aber von Interesse, durch einen einfachen Anzeiger feststellen zu können, ob es sich bei einer vorliegenden Lösung um eine 1-, 3-, 5-, 7-, 9- oder 11-normale Lauge handelt. Dies kann bisher selbst von einem pH-Meter nicht geleistet werden. Vielmehr muß man eine verhältnismäßig aufwendige Titration mit einer Säurelösung durchführen, um den Gehalt zu ermitteln.

Der Erfindung liegt die Aufgabe zugrunde, Indikatoren zur Verfügung zu stellen, mit denen die Bestimmung von Protonenkonzentrationen im stark alkalischen Bereich auf einfache Weise möglich ist.

Überraschenderweise wurde gefunden, daß bestimmte substantive Azofarbstoffe für den erfindungsgemäßen Zweck einsetzbar sind.

Gegenstand der Erfindung sind Indikatoren zur Bestimmung der Protonenkonzentration stark alkalischer wäßriger Lösungen, die dadurch gekennzeichnet sind, daß sie mindestens eine Verbindung der allgemeinen Formel (I) enthalten 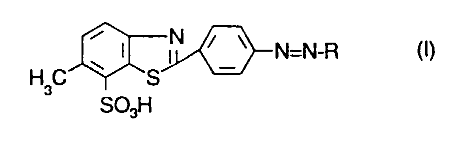 worin
- R =: 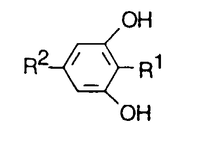 und
- R¹ =: H, OH, Alkyl mit 1-6 C-Atomen, Alkanoyl mit 2-7 C-Atomen,
- R² =: H, Alkyl mit 1-6 C-Atomen
bedeuten. Die Indikatoren sind vorzugsweise auf einen Träger aufgebracht.

Diese Verbindungen werden aus in der Farbstoffchemie bekannten Grundkörpern hergestellt, die den Farbstoffen erfahrungsgemäß substantiven Charakter verleihen. Die Grundkörper tragen mindestens eine Aminogruppe, die diazotiert und mit Phenolen zu den erfindungsgemäßen Indikatoren gekuppelt werden (Ullmann, Band A 3, 5. Auflage, 1985, Seiten 279-280). Die Bindungsstelle zwischen den diazotierten Grundkörpern und den Phenolen kann sich an allen nicht mit Substituenten besetzten Stellen der Phenole befinden.

Als besonders vorteilhaft hat sich 2-(4-Aminophenyl)6-methylbenzothiazol-7-sulfonsäure als Grundkörper erwiesen. Die Diazotierung und anschließende Kupplung des Grundkörpers erfolgt mit Phenolderivaten, vorzugsweise mit Pyrogallol, 3,5-Dihydroxytoluol und 2,6-Dihydroxyacetophenon.

Die Substanzen können in Lösung wie andere pH-Indikatoren eingesetzt werden. Vorteilhafter werden jedoch Materialien aus Cellulose, z.B. Papier, Folien oder Gewebe mit diesen Substanzen eingefärbt. Wegen ihrer Substantivität bleiben die Substanzen auf diesen Materialien verhältnismäßig fest haften und eignen sich so z.B. in Streifenform direkt zur Anzeige der Laugenkonzentration. In anderen Ausführungsformen wird das gefärbte Material auf einer farblosen oder weißen Kunststoffolie als Träger befestigt und kann so nicht nur visuell, sondern auch fotometrisch im Durchlicht oder in Remission ausgewertet werden.

Um die Laugenkonzentrationen messen zu können, muß vorher eine Farbvergleichsskala hergestellt werden. Für Kalilauge und beim direkten Einsatz des Indikators geht man wie folgt vor:
a) Es werden Kaliumhydroxidlösungen verschiedener Konzentrationen hergestellt, z.B. 1 N, 2N, 4N,... 18 N oder 10 %, 20 %, 30 % ... 100%.
b) In jeweils 100 ml dieser Lösungen wird 1,0 mg des Indikators aufgelöst.
c) Diese Lösungen werden in 10-mm-Küvetten gefüllt, die nebeneinander aufgestellt werden. Diese Reihe dient als Farbvergleichsskala.

In einer 100-ml-Probe, deren Konzentration an Kaliumhydroxid bestimmt werden soll, wird 1,0 mg des Indikators aufgelöst und die Lösung in eine 10-mm-Küvette gefüllt. Die Bestimmung der Laugenkonzentration erfolgt so, daß man die Farbe der Küvette mit der unbekannten Probe mit der Farbvergleichsskala vergleicht Da die Konzentrationen der Lösungen der Farbvergleichsskala bekannt sind, ist somit auch die Laugenkonzentration der unbekannten Probe bekannt.

### Beispiel 1

### Herstellung eines Indikators

38,4 g (120 mMol) 2-(4-Aminophenyl)-6-methyl-benzothiazol-7-sulfonsäure wurden in 75 ml 2 N Natronlauge und 300 ml Wasser gelöst. Zu dieser Lösung wurde bei Raumtemperatur innerhalb von 10 Minuten eine Lösung von 9,1 g Natriumnitrit in 75 ml Wasser getropft und weitere 30 Minuten gerührt. Anschließend wurden bei 0-5 °C 300 ml 1 N Salzsäure zugetropft und die einen orange-gelben Niederschlag enthaltende Lösung noch weitere 30 Minuten bei 0-5 °C gerührt.

Danach wurde das Reaktionsgemisch unter Stickstoff bei 5-10 °C in eine Lösung von 21,3 g 3,5-Dihydroxytoluol (150 mMol) in 27,8 ml 32%iger Natronlauge und 200 ml Ethanol getropft (20 min), wobei ein orange-roter Niederschlag anfiel. Es wurde eine Stunde bei 5-10 °C, dann weitere 18 Stunden bei 20-25 °C gerührt. Das Reaktionsgemisch wurde kurz erwärmt und unter Rühren langsam in 101 heißes i-Propanol getropft. Dabei fiel ein orange-roter Niederschlag aus, der abgesaugt und getrocknet wurde.

Ausbeute: 34,2 g (62,5 % der Theorie) orange bis rotbraune Kristalle.

### Beispiel 2

### Herstellung und Funktionsweise eines Teststäbchens

Es wurden folgende zwei Lösungen hergestellt:
1. 10 mg des Azofarbstoff nach Beispiel 1 in 50 ml Wasser.
2. 10 mg des Azofarbstoffs aus 2-(4-Aminophenyl)-6-methylbenzothiazol-7-sulfonsäure und 2,6-Dihydroxyacetophenon in 50 ml Waser.

In jede dieser Lösungen wurden 6 mm breite und 300 mm lange Filterpapierstreifen (z.B. Schleicher und Schüll Nr. 604) getaucht und anschließend getrocknet. Die so erhaltenen beiden Indikatorpapierstreifen wurden mit beidseitig klebendem Band auf eine 75 mm breite, 0,2 mm dicke und 300 mm lange weiße PVC-Folie geklebt. Die erste Zone steht dabei direkt am Rand der Folie. Der Abstand zwischen den beiden Zonen beträgt 3 mm. Das Folienband wurde anschließend in 6 mm breite Teststäbchen zerschnitten. Auf jedem Teststäbchen befinden sich dann zwei Testzonen, die im alkalischen Bereich folgende Farben zeigen:

| Kalilauge | Zone 1 | Zone 2 |
|---|---|---|
| 0.1 N | gelb | gelb |
| 0.3 N | gelb | orange |
| 0.5 N | gelb | rot |
| 1.0 N | gelb | rotviolett |
| 4.0 N | gelb | blauviolett |
| 8.0 N | orange | blauviolett |
| 10.0 N | hellrot | blauviolett |
| 12.0 N | dunkelrot | blauviolet |

Wie aus der Tabelle zu ersehen ist, dient im Bereich zwischen 0.1 und 4 N Kalilauge Zone 2, im Bereich zwischen 4 und 12 N Kalilauge Zone 1 als Indikator. Analoge Färbungen wurden mit anderen starken Basen wie Natronlauge oder Bariumhydroxidlösung erhalten, wenn die entsprechenden Konzentrationen eingestellt waren.

## Patentansprüche

1. Verbindungen der Formel (I) 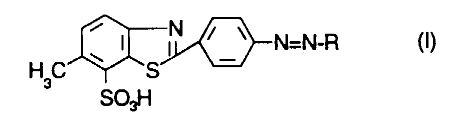 worin
R = 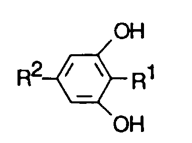 und
R¹ = H, OH, Alkyl mit 1-6 C-Atomen, Alkanoyl mit 2-7 C-Atomen,
R² = H, Alkyl mit 1-6 C-Atomen
bedeuten.

2. Indikatoren zur Bestimmung der Protonenkonzentration stark alkalischer wäßriger Lösungen, dadurch gekennzeichnet, daß sie mindestens eine Verbindung der allgemeinen Formel (I) nach Anspruch 1 enthalten.

3. Indikatoren nach Anspruch 2, dadurch gekennzeichnet, daß sie auf einen Träger aufgebracht sind.

## Claims

1. Compounds of the formula (I) 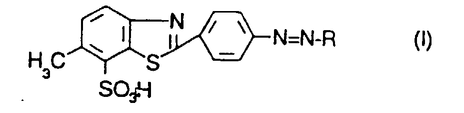 in which
R = 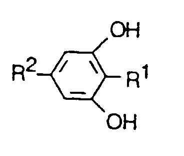 and
R¹ = H, OH, alkyl having 1-6 C atoms, alkanoyl having 2-7 C atoms,
R² = H, alkyl having 1-6 C atoms.

2. Indicators for determining the proton concentration of strongly alkaline aqueous solutions, characterized in that they contain at least one compound of the general formula (I) according to Claim 1.

3. Indicators according to Claim 2, characterized in that they are applied to a support.

## Revendications

1. Composés de formule (I) 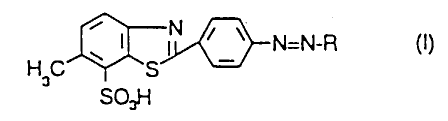 où
R = 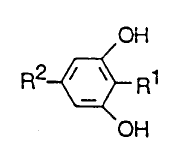
R¹ représentant H, OH, un alkyle comportant 1 à 6 atomes de C, un alcanoyle comportant 2 à 7 atomes de C,
R² H, un alkyle comportant 1 à 6 atomes de C.

2. Indicateurs permettant de déterminer la concentration en protons de solutions aqueuses fortement alcalines, caractérisés en ce qu'ils contiennent au moins un composé de formule générale (I) selon la revendication 1.

3. Indicateurs selon la revendication 2, caractérisés en ce qu'ils sont appliqués sur un support.
